# EUROPÄISCHE PATENTSCHRIFT

(11) **EP 2 300 118 B1**
(45) Veröffentlichungstag und Bekanntmachung des Hinweises auf die Patenterteilung: **19.10.2016**
(21) Anmeldenummer: 09769018.4
(22) Anmeldetag: 26.06.2009
(51) Int. Cl.: B01D 15/38

(54) **VERFAHREN ZUR PROTEINAUFREINIGUNG UNTER DENATURIERENDEN BEDINGUNGEN**
METHOD FOR PROTEIN PURIFICATION UNDER DENATURING CONDITIONS
PROCÉDÉ DE PURIFICATION DE PROTÉINES EN CONDITIONS DÉNATURANTES

(30) Priorität: 27.06.2008 DE 102008030142
(43) Veröffentlichungstag der Anmeldung: 30.03.2011
(73) Patentinhaber: Qiagen GmbH, 40724 Hilden (DE)
(72) Erfinder: DEUTSCHMANN, Thomas, 42349 Wuppertal (DE); SCHÄFER, Frank, 40627 Düsseldorf (DE); BLOCK, Helena, 40724 Hilden (DE)
(74) Vertreter: Roth, Carla
(86) Internationale Anmeldenummer: PCT/EP2009/004632
(87) Internationale Veröffentlichungsnummer: WO 2009/156169

(56) Entgegenhaltungen:
- WO-A1-2007/058584
- WO-A2-2005/058930
- US-A1- 2002 042 097
- US-A1- 2006 105 389
- "The QIAexpressionist: A Handbook for high-level expression and purification of 6xHis-tagged proteins; Fifth Edition" Juni 2003 (2003-06), Qiagen , XP002551628 , Seiten 1-128 Seite 80 Seite 90 Seite 113
- LIN P C ET AL: "Adsorption behaviors of recombinant proteins on hydroxyapatite-based immobilized metal affinity chromatographic adsorbents" JOURNAL OF THE CHINESE INSTITUTE OF CHEMICAL ENGINEERS, TAIPEI, TW, Bd. 39, Nr. 5, 1. September 2008 (2008-09-01), Seiten 389-398, XP023316872 ISSN: 0368-1653 [gefunden am 2008-07-29]

## Beschreibung

Die vorliegende Erfindung betrifft ein Verfahren zur Proteinaufreinigung sowie dafür geeignete Kits und Puffersysteme.

Zur Aufreinigung von Proteinen über immobilisierte Metallaffinitätschromatographie (im Folgenden: IMAC) unter denaturierenden Bedingungen werden üblicherweise entweder Guadiniumhydrochlorid-haltige Puffer (siehe Hochuli *et al.* 1988) oder hochkonzentrierte, harnstoffhaltige Pufferlösungen eingesetzt. Harnstoffhaltige Pufferlösungen bieten den Vorteil gegenüber Guadiniumhydrochlorid-haltigen Puffern, dass die mit harnstoffhaltigen Pufferlösungen aufgereinigten Proteine direkt auf SDS-Gelen analysiert werden können. Die optimale Bindung der Proteine an die Matrix, die Stringenz der Waschschritte, sowie die effiziente Elution des interessierenden Proteins hängen entscheidend vom pH-Wert der einzelnen Pufferlösungen ab.

Die im Stand der Technik bekannten harnstoffhaltigen Pufferlösungen verlieren jedoch ihre Pufferwirkung über die Zeit, insbesondere, da ihr pH-Wert ansteigt. Dadurch wird die Stringenz der Waschschritte und somit die Selektivität der Proteinaufreinigung herabgesetzt. Aufgrund des höheren pH-Wertes werden Proteine unspezifisch an die Matrix gebunden und können nicht mehr effizient ausgewaschen werden. Auch nimmt die Elutionseffizienz ab. Zur Aufreinigung His-getaggter rekombinanter Proteine über IMAC unter denaturierenden Bedingungen müssen daher die verwendeten harnstoffhaltigen Pufferlösungen vor jeder Verwendung frisch angesetzt oder ihr pH-Wert muss korrigiert, d.h. neu eingestellt werden. Das ist zeitaufwendig und entsprechend nachteilig. Ein entsprechendes Verfahren ist bspw. in dem Handbuch "The Qiaexpressionist: A Handbook for high-level expression and purification of 6xHis-tagged proteins; Fifth Edition" offenbart. Hier muss der Benutzer die Anwendungspuffer frisch ansetzen und deren pH Wert vor der Anwendung einstellen. Entsprechende oder ähnliche Verfahren wurden auch weitreichend im Stand der Technik zur Aufreinigung von Proteinen eingesetzt (siehe bspw. US 2006/105389, US 2002/042097 und WO 2005/058930).

Davon ausgehend ist es Aufgabe der vorliegenden Erfindung, ein verbessertes Verfahren zur Proteinaufreinigung bereitzustellen.

Die Lösung der oben gestellten Aufgabe ergibt sich durch die in den Ansprüchen 1 bis 16 beschriebenen Verfahren, Kits und Puffersysteme.

Gemäß einer Ausführungsform der Erfindung wird ein Verfahren zur Herstellung eines Anwendungspuffers zur Aufreinigung von Proteinen über IMAC unter denaturierenden Bedingungen bereitgestellt, welches dadurch gekennzeichnet ist, dass eine definierte Menge eines Pufferkonzentrates mit definiertem pH-Wert, das keine störenden Mengen an Harnstoff aufweist, mit einer definierten Menge eines Harnstoffkonzentrates gemischt wird, wodurch ein Anwendungspuffer mit definiertem pH-Wert bereitgestellt wird.

Im Zusammenhang mit der vorliegenden Erfindung werden die Begriffe "Pufferkonzentrat" und "Anwendungspuffer" verwendet. Der Begriff "Pufferkonzentrat" bezieht sich auf Pufferlösungen, die nicht direkt zur Aufreinigung eingesetzt werden, sondern erst mit Harnstoff vermischt werden, um den eigentlichen Anwendungspuffer zu erhalten. Das Pufferkonzentrat weist keine störenden Mengen an Harnsstoff auf. Die Pufferkonzentrate weisen vorzugsweise keinerlei Harnstoff auf. Der Begriff "Anwendungspuffer" wird für Pufferlösungen verwendet, welche bereits mit Harnstoff gemischt und direkt zur Proteinaufreinigung eingesetzt werden können, bspw. als Binde-, Wasch- oder Elutionspuffer.

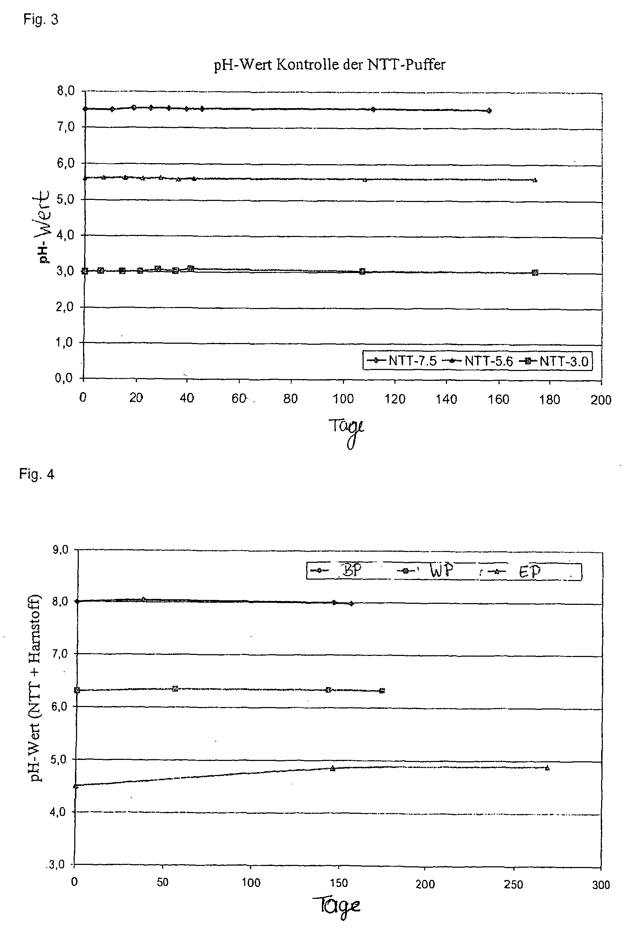

Kern der vorliegenden Erfindung ist die separate Bereitstellung der zur Proteinaufreinigung notwendigen Komponenten, die miteinander reagieren und so zu einem Anstieg des pH-Wertes führen können. Erfindungsgemäß wird wenigstens ein Pufferkonzentrat mit definiertem und entsprechend voreingestelltem pH-Wert separat von dem Harnstoffkonzentrat bereitgestellt. Eine definierte Menge des Harnstoffkonzentrats wird erst kurz vor der Anwendung mit einer definierten Menge des Pufferkonzentrats gemischt, wodurch ein Anwendungspuffer mit definierter Zusammensetzung und definiertem und entsprechend gewünschtem pH-Wert erhalten wird. Der Vorteil der separaten Bereitstellung der Konzentrate liegt darin, dass die Einzelkomponenten (Pufferkonzentrat und Harnstoffkonzentrat) lagerungsstabil sind und der pH-Wert der separat gelagerten Konzentrate über einen langen Zeitraum konstant bleibt (siehe auch Fig. 3). Durch die Vermischung von nur zwei Komponenten, nämlich des Pufferkonzentrats mit dem Harnstoffkonzentrat, kann selbst nach langer Lagerung der Konzentrate ein Anwendungspuffer mit definierter Zusammensetzung und definiertem pH-Wert erzeugt werden (siehe Fig. 4). Bei dem erfindungsgemäßen Verfahren ist im Unterschied zum Stand der Technik daher kein erneutes Einstellen des pH-Wertes vor oder während der Proteinaufreinigung notwendig. Auch entfällt ein vollständiger Neuansatz der Anwendungspuffer. Nach Mischen der Konzentrate ist der Anwendungspuffer direkt einsetzbar. Für den Anwender ist die Verwendung entsprechender "ready-to-use" Reagenzien insbesondere in einem Kit-Format arbeitserleichternd und zeitsparend. Da erfindungsgemäß immer ein Anwendungspuffer gleicher Zusammensetzung und mit definiertem (gleichem) pH-Wert erzeugt wird, sind die Aufreinigungsergebnisse reproduzierbar und von gleich bleibender Qualität. pH-Wert bedingte Schwankungen der Anwendungspuffer und damit verbundene Fehlerquellen werden vermieden. Das erfindungsgemäße Verfahren ist nicht nur im manuellen Format anwenderfreundlich, sondern zudem automatisierbar, da lediglich die Komponenten miteinander gemischt werden müssen, jedoch keine Feinjustierung oder ein Neuansatz der Anwendungspuffer erforderlich ist. Eine Automatisierung war mit den im Stand der Technik bekannten Puffersystemen und Verfahren schlecht möglich. Aufgrund der fehlenden pH-Stabilität waren ferner auch keine lagerungsfähigen, geschlossenen Kit-Konzepte (bei denen keine Änderungen des Anwenders an den Puffern vorgenommen werden) möglich, da der Anwender Veränderungen an den bereitgestellten Puffern vornehmen musste (bspw. pH-Wert Einstellung).

Das erfindungsgemäße Verfahren findet insbesondere Anwendung in der Aufreinigung His-getaggter Proteine mittels der Ni²⁺-NTA (Nickel-Nitrilotriessigsäure)-Chromatographie.

Gemäß einer Ausführungsform des erfindungsgemäßen Verfahrens werden drei Pufferkonzentrate bereitgestellt, die jeweils unterschiedliche, bereits voreingestellte pH-Werte aufweisen. Die Zusammensetzung der Pufferkonzentrate kann gleich oder verschieden sein. Durch Mischen dieser Konzentrate mit einer definierten Menge Harnstoff erhält man wenigstens drei Anwendungspuffer mit jeweils unterschiedlichem pH-Wert. Die drei grundlegenden Schritte des Verfahrens, Bindung an die Matrix, Waschen der Matrix und die anschließende Elution des interessierenden Proteins von der Matrix benötigen jeweils Puffer mit speziell angepassten, d.h. eingestellten pH-Werten (siehe oben). Durch die Bereitstellung von wenigstens drei Pufferkonzentrate mit unterschiedlichem pH-Wert werden durch Mischung mit einer definierten Menge an Harnstoffkonzentrat wenigstens drei Anwendungspuffer hergestellt, die nicht nur eine definierte Zusammensetzung sondern auch definierte und für den jeweiligen Schritt angemessene, bzw. erforderliche pH-Werte besitzen.

Diese Anwendungspuffer sind direkt, d.h. ohne weitere Modifizierungen durch den Anwender bzw. in einem automatisierten Verfahren einsetzbar.

Gemäß einer Ausführungsform des erfindungsgemäßen Verfahrens weist das Pufferkonzentrat, welches zur Herstellung des Anwendungspuffers zur Bindung eingesetzt wird, einen alkalischen pH-Wert auf, bevorzugt einen pH-Wert von 7,0 bis 9,0, besonders bevorzugt einen pH-Wert von ca. 7,5. Durch Mischung dieses Pufferkonzentrates mit einer definierten Menge des Harnstoffkonzentrates wird ein Anwendungspuffer mit gleichem oder höherem pH-Wert erzeugt. Gemäß einer Ausführungsform weist der Anwendungspuffer zur Bindung einen alkalischen pH-Wert auf, bevorzugt einen pH-Wert von 7,0 bis 9,0, besonders bevorzugt einen pH-Wert von ca. 8. Der Bindepuffer kann auch als Lysepuffer eingesetzt werden und umgekehrt.

Das Pufferkonzentrat, welches zur Herstellung des Anwendungspuffers zum Waschen der Matrix eingesetzt wird, weist gemäß dieser Ausführungsform einen leicht sauren pH-Wert auf, bevorzugt einen pH-Wert von 5,0 bis 7,0, besonders bevorzugt einen pH-Wert von ca. 5,6. Durch Mischung dieses Pufferkonzentrates mit einer definierten Menge des Harnstoffkonzentrates wird ein Anwendungspuffer mit höherem pH-Wert erzeugt. Gemäß einer Ausführungsform weist der Anwendungspuffer zum Waschen der Matrix und zum Lösen unspezifisch gebundener Proteine einen leicht sauren bis neutralen pH-Wert auf, bevorzugt einen pH-Wert von 5,5 bis 7,0, besonders bevorzugt einen pH-Wert von 6 bis 6,5.

Das Pufferkonzentrat, welches zur Herstellung des Anwendungspuffers zur Elution des Proteins eingesetzt wird, weist gemäß einer Ausführungsform einen sauren pH-Wert auf, bevorzugt einen pH-Wert weniger als 4, besonders bevorzugt einen pH-Wert von weniger als 3,5. Durch Mischung dieses Pufferkonzentrates mit einer definierten Menge des Harnstoffkonzentrates wird ein Anwendungspuffer mit höherem pH-Wert erzeugt. Gemäß einer Ausführungsform weist der Anwendungspuffer zur Elution des aufzureinigenden Proteins aus der Matrix einen sauren pH-Wert auf, bevorzugt einen pH-Wert von unter 5,5, besonders bevorzugt einen pH-Wert von unter 5 bspw. bei ca. 4,5.

Die pH-Werte dieser Pufferkonzentrate bleiben über lange Zeit stabil, wie aus Fig. 3 zu ersehen ist. Ein wichtiger Grund für diese pH-Stabilität gegenüber den im Stand der Technik bekannten Pufferlösungen ist die Abwesenheit von störenden Mengen an Harnstoff in den Pufferkonzentraten. Die Anwendungspuffer erreichen durch die gezielte Mischung bestimmter Mengen an Pufferkonzentrat mit Harnstoffkonzentrat eine vorher bestimmbare, bzw. definierte Zusammensetzung und einen vorher bestimmbaren, bzw. definierte pH-Wert. Durch diese pH-Stabilität und Konstanz in der Zusammensetzung werden die Ergebnisse des Proteinaufreinigungsverfahrens reproduzierbar und qualitativ vergleichbar. Da der Anwender die Puffer nicht selbst ansetzen muss, werden Schwankungen in der Zusammensetzung vermieden. Die pH-Werte der aus den Konzentraten erzeugten Anwendungspuffer bleiben selbst bei langer Lagerung der Konzentrate konstant, wie es aus Fig. 4 zu ersehen ist.

Die Pufferkonzentrate weisen vorzugsweise einen biologischen Puffer auf. Biologische Puffer werden in vielfältiger Form im Bereich der Biologie und Molekularbiologie eingesetzt. Im Unterschied zu klassischen, anorganischen puffernden Substanzen beeinflussen sie das zu untersuchende System nicht nachteilig. Biologische Puffer enthalten oftmals Zwitterionen. Eine Übersicht über biologische Puffer findet sich bspw. auf der der Homepage von Sigma-Aldrich (www.sigmaaldrich.com).

Gemäß einer Ausführungsform weist das Pufferkonzentrat zur Herstellung des Binde-Anwendungspuffers einen Puffer auf, der eine Pufferkapazität im Alkalischen aufweist, vorzugsweise in einem Bereich von ca. 7,0 bis 9,0. HEPES (2-(4-(2-Hydroxyethyl)- 1-piperazinyl)-ethansulfonsäure), MES (2-(N-Morpholino)ethansulfonsäure), Natriumphosphat-, Citrat-, Succinat- oder Acetatpuffer seien hier als mögliche, erfindungsgemäß einsetzbare Puffer erwähnt. Bevorzugt werden aminhaltige Puffer eingesetzt. Ein bevorzugtes Beispiel für einen biologischen, aminhaltigen Puffer ist ein Tris-Puffer.

Gemäß einer Ausführungsform weisen die Pufferkonzentrate zur Herstellung des Wasch- und Elutions- Anwendungspuffers einen Puffer auf, der eine Pufferkapazität im Neutralen bis Sauren aufweist, vorzugsweise in einem Bereich von ca. 4,0 bis 7,5. Citrat-, Succinat- oder Acetatpuffer sind hier erfindungsgemäß geeignete Puffer. Ein besonders geeignetes Beispiel für einen biologischen Puffer ist Bis-Tris.

Die Pufferkonzentrate zur Herstellung des Waschpuffers und des Elutionspuffers können die gleiche Zusammensetzung aufweisen, wobei sie jedoch einen unterschiedlichen pH-Wert aufweisen. Der pH-Wert des Waschkonzentrates ist höher als der des Elutionskonzentrates.

Gemäß einer Ausführungsform weisen die Pufferkonzentrate ferner Salze, beispielsweise NaCI, KCl oder KH₂PO₄, vorzugsweise NaH₂PO₄ und/oder nicht-ionische Tenside, beispielsweise Triton X100, Triton X114, NP-40, CHAPS, DDM, b-OG, NG, Brij35 oder Digitonin, oder vorzugsweise Tween auf. Gemäß einer Ausführungsform weisen die Pufferkonzentrate zusätzlich ein Konservierungsmittel, vorzugsweise Na-N₃, auf.

Gemäß einer Ausführungsform des erfindungsgemäßen Verfahrens enthält das zur Herstellung des Anwendungspuffers verwendete Pufferkonzentrat 25-500 mM, vorzugsweise 50-300 mM, besonders bevorzugt 100-250 mM Tris(hydroxymethyl)-aminomethan (insbesondere zur Herstellung des Bindepuffers) oder BisTris(Bis(2-hydroxyethyl)amino-tris(hydroxymethyl)methan (insbesondere zur Herstellung des Wasch- bzw. Elutionspuffers), sowie zusätzlich 125-750 mM, vorzugsweise 250 bis 500 mM, besonders bevorzugt 300-450 mM NaH₂PO₄ und/oder 0,025 bis 0,5 % (v/v), vorzugsweise 0,05-0,3 % (v/v), besonders bevorzugt 0,1 bis 0,25 % (v/v) nichtionische Tenside und/oder 0,0025 bis 0,05%, vorzugsweise 0,005-0,03%, besonders bevorzugt 0,01 bis 0,025% NaN₃. Bevorzugt wird Tris(hydroxymethyl)-aminomethan oder BisTris(Bis(2-hydroxyethyl)amino-tris(hydroxymethyl)methan in Form von Tris(hydroxymethyl)-aminomethanchlorid oder BisTris(Bis(2-hydroxyethyl)amino-tris(hydroxymethyl)methanchlorid eingesetzt.

Gemäß einer Ausführungsform des erfindungsgemäßen Verfahrens ist das zur Herstellung des Anwendungspuffers separat bereitgestellte Harnstoffkonzentrat eine wässrige Lösung mit 8 bis 10 M Harnstoff; gemäß einer bevorzugten Ausführungsform ist das Harnstoffkonzentrat eine wässrige Lösung mit 9,3 bis 9,8 M Harnstoff, vorzugsweise 9,6 M Harnstoff.

Gemäß einer Ausführungsform wird ein Teil Pufferkonzentrat mit 3,7 Teilen Harnstoffkonzentrat gemischt, um einen erfindungsgemäßen Anwendungspuffer zu erhalten. Gemäß einer Ausführungsform wird dieses Mischungsverhältnis auf die Herstellung aller Anwendungspuffer angewandt.

Die Erfindung betrifft ferner die Verwendung eines Harnstoffkonzentrats zur Herstellung eines Anwendungspuffers mit definiertem pH-Wert, wobei eine definierte Menge eines Harnstoffkonzentrats mit einer definierten Menge eines Pufferkonzentrats gemischt wird, wobei das Pufferkonzentrat keine störenden Mengen an Harnstoff aufweist. Durch die separate Bereitstellung des Harnstoffkonzentrats und seine Mischung mit dem Pufferkonzentrat kurz vor der Anwendung werden Anwendungspuffer erzielt, welche nicht nur eine definierte Zusammensetzung, sondern auch einen bestimmten, vorher definierten pH-Wert aufweisen. Dies trägt zu besseren und vor allem standardisierbaren Bedingungen in der Proteinaufreinigung bei. Einzelheiten zu den Vorteilen, der Zusammensetzung der Harnstoff- und Pufferkonzentrate wurden oben im Detail erläutert. Wir verweisen auf unsere obige Offenbarung, die auch im Zusammenhang mit der Verwendung gilt.

Gemäß einer besonderen Ausführungsform wird ferner ein Kit zur Aufreinigung von Proteinen über IMAC unter denaturierenden Bedingungen bereitgestellt, welches mindestens ein Pufferkonzentrat mit definiertem pH-Wert und ein Harnstoffkonzentrat zur Herstellung wenigstens eines Anwendungspuffers mit definiertem pH-Wert sowie eine IMAC Matrix aufweist. Das Pufferkonzentrat weist keine störenden Mengen an Harnstoff auf. Das Harnstoffkonzentrat ist eine wässrige Lösung mit 8 bis 10M Harnstoff.

Gemäß einer Ausführungsform weist das Kit wenigstens drei Pufferkonzentrate mit jeweils unterschiedlichem, definiertem pH-Wert auf, die durch Mischung mit jeweils einer definierten Menge an Harnstoffkonzentrat zu wenigstens drei verschiedenen Anwendungspuffer mit definierter Zusammensetzung und definiertem pH-Wert führen.

Das erfindungsgemäße Kit ist aufgrund der separat bereitgestellten Konzentrate stabil und damit in vorteilhafter Weise lagerungsfähig. Durch die wenigen und direkt einsatzfähigen Komponenten ist das Kit übersichtlich gestaltet und für den Anwender sicher und schnell einsetzbar. Es sind keine Einstellungen der Pufferbedingungen durch den Anwender erforderlich, wodurch reproduzierbare und qualitativ gleichbleibende Ergebnisse erzeugt werden. Auch kann das Kit durch weniger geschultes Personal eingesetzt werden, da die Fehleranfälligkeit reduziert ist. Es ist dadurch besonders für einen hohen Probendurchsatz geeignet. Ferner kann das Kit in Kombination mit automatisierten Verfahren eingesetzt werden.

Das Kit kann auch ein Puffersystem zur Aufreinigung von Proteinen über IMAC unter denaturierenden Bedingungen darstellen, welches mindestens ein Pufferkonzentrat mit definiertem pH-Wert und ein Harnstoffkonzentrat aufweist, welches durch Mischung mit dem Pufferkonzentrat zu einem Anwendungspuffer mit definiertem pH-Wert führt.

Einzelheiten zu den Pufferkonzentraten, dem Harnstoffkonzentrat sowie den daraus erzeugten Anwendungspuffern sind oben im Detail beschrieben. Wir verweisen auf unsere obigen Ausführungen, die auch im Zusammenhang mit dem Kit gelten und Bestandteile der entsprechenden Offenbarung sind.

Gemäß einer Ausführungsform des erfindungsgemäßen Kits enthält das zur Herstellung des Anwendungspuffers verwendete Pufferkonzentrat 25-500 mM, vorzugsweise 50-300 mM, besonders bevorzugt 100-250 mM Tris(hydroxymethyl)-aminomethan (insbesondere zur Herstellung des Bindepuffers) oder BisTris(Bis(2-hydroxyethyl)amino-tris(hydroxymethyl)methan (insbesondere zur Herstellung des Wasch- bzw. Elutionspuffers), sowie zusätzlich 125-750 mM, vorzugsweise 250 bis 500 mM, besonders bevorzugt 300-450 mM NaH₂PO₄ und/oder 0,025 bis 0,5 % (v/v), vorzugsweise 0,05-0,3 % (v/v), besonders bevorzugt 0,1 bis 0,25 % (v/v) nichtionische Tenside und/oder 0,0025 bis 0,05%, vorzugsweise 0,005-0,03%, besonders bevorzugt 0,01 bis 0,025% NaN₃. Bevorzugt wird Tris(hydroxymethyl)-aminomethan oder BisTris(Bis(2-hydroxyethyl)amino-tris(hydroxymethyl)methan in Form von Tris(hydroxymethyl)-aminomethanchlorid oder BisTris(Bis(2-hydroxyethyl)amino-tris(hydroxymethyl)methanchlorid eingesetzt.

Gemäß einer Ausführungsform des erfindungsgemäßen ist das Harnstoffkonzentrat eine wässrige Lösung mit 8 bis 10 M Harnstoff; gemäß einer bevorzugten Ausführungsform ist das Harnstoffkonzentrat eine wässrige Lösung mit 9,3 bis 9,8 M Harnstoff, vorzugsweise 9,6 M Harnstoff.

Gemäß einer Ausführungsform verändern die Kitkomponenten (Pufferkonzentrat(e), Harnstoffkonzentrat) ihren pH-Wert für wenigstens 100, vorzugsweise wenigstens 150 Tage um nicht mehr als +/- 0,5 vorzugsweise um +/- 0,3 bzw. +/- 0,1. Sie sind daher lagerungsfähig und als geschlossenes Kit-Konzept einsetzbar.

Die lagerungsfähigen Kitkomponenten erzeugen vorzugsweise nach einer Lagerungsdauer von wenigstens 100, vorzugsweise 150 Tagen bei Mischung der Pufferkonzentrate mit dem Harnstoffkonzentrat Anwendungspuffer mit definiertem pH-Wert +/- 0,5.

Das Kit kann ferner eine IMAC Matrix aufweisen, vorzugsweise eine Ni²⁺-NTA Matrix. Diese kann auch in Form von Beads, insbesondere magnetischen Partikeln, bereitgestellt werden.

### FIGUREN

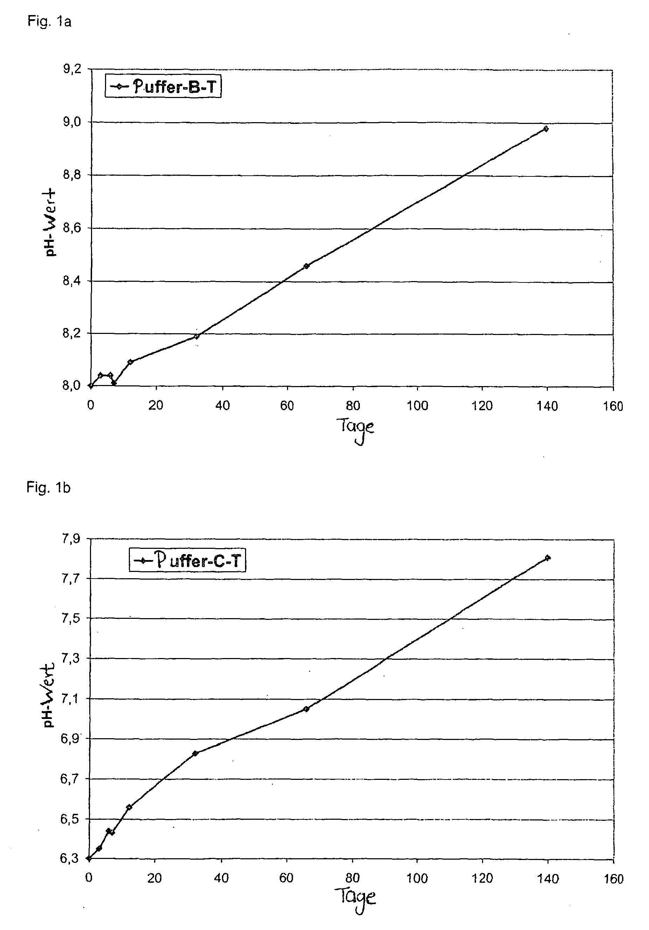

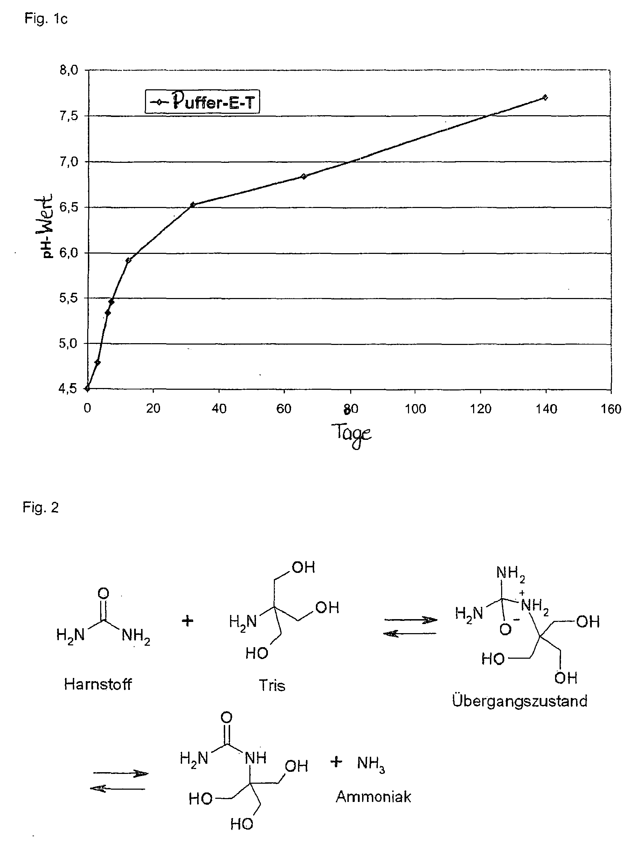

**Fig. 1****:** Demonstriert sind die Probleme der fehlenden pH-Stabilität von im Stand der Technik bekannten Puffersystemen, die standardmäßig zur denaturierenden Aufreinigung von 6x Hisgetaggten Proteine eingesetzt werden. Folgende Pufferzusammensetzungen wurden für den Binde-, Wasch- und Elutionspuffer (Anwendungspuffer) genutzt.
   100mM NaH₂PO₄, 10mM Tris, 8M Harnstoff und 0,05% Tween 20. Der pH-Wert des BindePuffers (Buffer B-T) wurde auf pH 8,0, der des Waschpuffers (Buffer C-T) auf pH 6,3 und der des Elutions-Puffers (Buffer E-T) auf pH 4,5 eingestellt. Wie Fig. 1 a) bis c) zeigt, steigt der pH-Wert der einzelnen Pufferlösungen bereits nach wenigen Tagen linear an.
   Fig. 1 a) zeigt die Entwicklung des pH-Wertes für eine harnstoffhaltige Binde-Pufferlösung "Buffer B-T" zur Bindung des Proteins an die Matrix. Wie in der Grafik deutlich sichtbar ist, steigt der zunächst auf pH 8,0 eingestellte pH-Wert der Pufferlösung nach wenigen Tagen linear an und erreicht nach ca. zwei Monaten einen pH-Wert von 8,4.
   Fig. 1 b) zeigt die Entwicklung des pH-Wertes für einen harnstoffhaltigen Anwendungspuffer "Buffer C-T" zum Waschen und Lösen unspezifisch gebundener Proteine aus der Matrix. Wie in der Grafik deutlich sichtbar ist, steigt der zunächst auf pH 6,3 eingestellte pH-Wert der Pufferlösung schon nach wenigen Tagen linear an und erreicht nach ca. zwei Monaten bereits einen neutralen pH-Wert von 7. Ein effizientes Waschen der Matrix ist unter diesen Bedingungen nicht mehr gegeben, der pH-Wert muss neu eingestellt werden.
   Fig. 1 c) zeigt Entwicklung des pH-Wertes für einen harnstoffhaltigen Anwendungspuffer "Buffer E-T" zur Elution der interessierenden Proteine aus der Matrix. Wie in der Grafik deutlich sichtbar ist, steigt der zunächst auf pH 4,5 eingestellte pH-Wert der Pufferlösung schon nach wenigen Tagen linear an und erreicht nach ca. zwei Monaten bereits einen pH-Wert von über 6,5. Hier ist keine effiziente Elution des Proteins mehr möglich, der pH-Wert muss neu eingestellt werden.
   Durch den pH-Anstieg ist die Bindung des Proteins an die Matrix nicht mehr optimal; zudem leidet die Stringenz der Waschschritte und die Elutionseffizienz ist herabgesetzt. Beim Einsatz dieser Puffer über längere Zeit wäre eine regelmäßige pH-Korrektur notwendig.
**Fig. 2****:** Der Anstieg der pH-Werte der harnstoffhaltigen Pufferlösungen könnte auf der hier dargestellten Reaktionsabfolge beruhen. Harnstoff könnte mit Tris zu einer Übergangsverbindung reagieren, aus der sich dann Ammoniak, eine starke Base abspaltet.
   Durch die Entstehung von Ammoniak ändert sich dann der pH-Wert der Pufferlösung schnell zum Basischen hin. Durch die Reaktion des Harnstoffs mit Tris gehen ferner Trismoleküle verloren, die dann nicht mehr für eine puffernde Wirkung zu Verfügung stehen.
**Fig. 3****:** Demonstriert wird die pH-Wert Stabilität der erfindungsgemäßen Pufferkonzentrate über die Zeit. Es wurden acht pH-Messungen zu unterschiedlichen Zeitpunkten durchgeführt. Folgende Zusammensetzungen wurden für die Pufferkonzentrate gewählt: 370 mM NaH₂PO₄, 185 mM TrisCl, 0,185% (v/v) Tween 20, 0,02% NaN₃ für das Binde-Pufferkonzentrat; 370 mM NaH₂PO₄, 185 mM Bis- TrisCl, 0,185% (v/v) Tween 20, 0,02% NaN₃ für das Wasch- und das Binde-Pufferkonzentrat.
   Das auf pH 7,5 voreingestellte Binde-Pufferkonzentrat (hier NTT-7.5) behält seinen pH-Wert ohne Schwankungen auch noch nach mehr als 100 Tagen bei. Das auf pH 5,6 voreingestellte Wasch-Pufferkonzentrat (hier NTT-5.6) behält seinen pH-Wert ebenfalls ohne Schwankungen oder nennenswerte Änderungen des pH-Wertes auch noch nach mehr als 100 Tagen bei. Das auf pH 3,0 voreingestellte Binde-Pufferkonzentrat (hier NTT-3.0) behält seinen pH-Wert ohne nennenswerte Schwankungen auch noch nach mehr als 100 Tagen bei. Dieser Versuch belegt die pH-Stabilität der erfindungsgemäß eingesetzten Konzentrate, wodurch diese aufgrund ihrer Lager-Stabilität auch in geschlossenen Kit-Systemen zum Einsatz kommen können.
**Fig. 4****:** Gezeigt ist ein Beispiel für die pH-Stabilität der erfindungsgemäß aus den Konzentraten (Pufferkonzentrat und Harnstoffkonzentrat) hergestellten Anwendungspuffer (BP = Bindepuffer; WP = Waschpuffer; EP = Elutionspuffer). Für die Herstellung der Anwendungspuffer wurden die Pufferkonzentrate mit den in Fig. 3 beschriebenen Zusammensetzungen und pH-Werten gewählt. Das Harnstoffkonzentrat bestand aus einer wässrigen 9,6 M Harnstofflösung, wodurch die fertigen Anwendungspuffer 7,0 M Harnstoff aufwiesen.
   Der Anwendungspuffer zur Bindung (pH 8,0) entsteht durch Mischen einer definierten Menge an Binde-Pufferkonzentrat mit pH 7,5 (siehe oben) mit dem Harnstoffkonzentrat. Der Anwendungspuffer zum Waschen (pH 6,3) entsteht durch die Mischung des Wasch-Pufferkonzentrats (siehe oben) mit dem Harnstoffkonzentrat. Die Anwendungspuffer wurden jeweils vor der Messung aus den gelagerten Konzentraten (Pufferkonzentrat und Harnstoffkonzentrat) gemischt. Die aus den Konzentraten erzeugten Anwendungspuffer zur Bindung und zum Waschen zeigen keine nennenswerte pH-Wert Änderung bis zum 150. Tag. Entsprechendes gilt für den Elutionspuffer.
   Der Anwendungspuffer zur Elution (pH 4,5) entsteht durch Mischen einer definierten Menge an Pufferkonzentrat mit pH 3,0 (siehe oben) mit dem Harnstoffkonzentrat. Über einen Mess-Zeitraum von mehr als 250 Tagen zeigt das Elutions-Pufferkonzentrat nur einen geringen, tolerierbaren Anstieg des pH-Wertes von 4,5 auf ca. 4,9. Dies zeigt, dass auch nach längerer Lagerung aus den einzelnen Konzentraten durch einfache Mischung Anwendungspuffer mit definierter Zusammensetzung und definiertem, gewünschtem pH-Wert erzielt werden. Die Vorteile wurden oben ausführlich dargelegt.

## Patentansprüche

1. Verfahren zur Herstellung eines Anwendungspuffers zur Aufreinigung von Proteinen über immobilisierte Metallaffinitätschromatographie (IMAC) unter denaturierenden Bedingungen, **dadurch gekennzeichnet, dass** eine definierte Menge eines Pufferkonzentrates mit definiertem pH-Wert, das keine störenden Mengen an Harnstoff aufweist, mit einer definierten Menge eines Harnstoffkonzentrates gemischt wird, wodurch ein Anwendungspuffer mit definiertem pH-Wert bereitgestellt wird.

2. Verfahren nach Anspruch 1, **dadurch gekennzeichnet, dass** wenigstens drei Pufferkonzentrate mit jeweils unterschiedlichem pH - Wert mit einer definierten Menge eines Harnstoffkonzentrates gemischt werden, wodurch drei Anwendungspuffer mit jeweils unterschiedlichem, definiertem pH-Wert bereitgestellt werden.

3. Verfahren nach Anspruch 1 oder 2, **gekennzeichnet durch** eines oder mehrere der folgenden Merkmale:
a. dass das Pufferkonzentrat, welches nach Mischung mit dem Harnstoffkonzentrat ein Anwendungspuffer zur Bindung ist, einen alkalischen pH-Wert aufweist; und/oder
b. dass das Pufferkonzentrat, welches nach Mischung mit dem Harnstoffkonzentrat ein Anwendungspuffer zur Bindung ist, einen pH-Wert von 7,0 bis 9,0 aufweist; und/oder
c. dass das Pufferkonzentrat, welches nach Mischung mit dem Harnstoffkonzentrat ein Anwendungspuffer zur Bindung ist, einen pH-Wert von ca. 7,5 aufweist; und/oder
d. dass das Pufferkonzentrat, welches nach Mischung mit dem Harnstoffkonzentrat ein Anwendungspuffer zum Waschen ist, einen leicht sauren pH-Wert aufweist; und/oder
e. dass das Pufferkonzentrat, welches nach Mischung mit dem Harnstoffkonzentrat ein Anwendungspuffer zum Waschen ist, einen pH-Wert von 5,5 bis 7,0 aufweist; und/oder
f. dass das Pufferkonzentrat, welches nach Mischung mit dem Harnstoffkonzentrat ein Anwendungspuffer zum Waschen ist, einen pH-Wert von ca. 5,6 aufweist; und/oder
g. dass das Pufferkonzentrat, welches nach Mischung mit dem Harnstoffkonzentrat ein Anwendungspuffer zur Elution ist, einen sauren pH-Wert aufweist; und/oder
h. dass das Pufferkonzentrat, welches nach Mischung mit dem Harnstoffkonzentrat ein Anwendungspuffer zur Elution ist, einen pH-Wert von unter 4, vorzugsweise unter 3,5 aufweist; und/oder
i. die Pufferkonzentrate weisen keinerlei Harnstoff auf.

4. Verfahren nach einem der Ansprüche 1 bis 3, **gekennzeichnet durch** eines oder mehrere der folgenden Merkmale
a. dass der **durch** Mischung des Pufferkonzentrates mit dem Harnstoffkonzentrat erzeugte Anwendungspuffer zur Bindung einen alkalischen pH-Wert aufweist; und/oder
b. dass der **durch** Mischung des Pufferkonzentrates mit dem Harnstoffkonzentrat erzeugte Anwendungspuffer zur Bindung einen pH-Wert von 7,0 bis 9,0 aufweist; und/oder
c. dass der **durch** Mischung des Pufferkonzentrates mit dem Harnstoffkonzentrat erzeugte Anwendungspuffer zur Bindung einen pH-Wert von ca. 8 aufweist; und/oder
d. dass der **durch** Mischung des Pufferkonzentrates mit dem Harnstoffkonzentrat erzeugte Anwendungspuffer zum Waschen einen leicht sauren bis neutralen pH-Wert aufweist; und/oder
e. dass der **durch** Mischung des Pufferkonzentrates mit dem Harnstoffkonzentrat erzeugte Anwendungspuffer zum Waschen einen pH-Wert von 5,5 bis 9,0 aufweist; und/oder
f. dass der **durch** Mischung des Pufferkonzentrates mit dem Harnstoffkonzentrat erzeugte Anwendungspuffer zum Waschen einen pH-Wert von 6 bis 6,5 aufweist; und/oder
g. dass der **durch** Mischung des Pufferkonzentrates mit dem Harnstoffkonzentrat erzeugte Anwendungspuffer zur Elution einen sauren pH-Wert aufweist; und/oder
h. dass der **durch** Mischung des Pufferkonzentrates mit dem Harnstoffkonzentrat erzeugte Anwendungspuffer zur Elution einen pH-Wert von unter 5,5, vorzugsweise unter 5 aufweist.

5. Verfahren nach einem oder mehreren der Ansprüche 1 bis 4, **dadurch gekennzeichnet, dass** das Pufferkonzentrat mit definiertem pH-Wert einen biologischen Puffer aufweist.

6. Verfahren nach einem oder mehreren der Ansprüche 3 bis 5, **dadurch gekennzeichnet,**
a. **dass** das Pufferkonzentrat zur Herstellung des Binde-Anwendungspuffer einen Puffer aufweist, der eine Pufferkapazität im Alkalischen aufweist, vorzugsweise Tris; und
b. **dass** die Pufferkonzentrate zur Herstellung des Wasch- und **Elutionsanwendungspuffers** einen Puffer aufweisen, der eine Pufferkapazität im Neutralen bis Sauren aufweist, vorzugsweise Bis-Tris; und/oder
c. **dass** die Pufferkonzentrate Salze, vorzugsweise NaH₂PO₄ und/oder Detergenzien, vorzugsweise Tween und/oder ein Konservierungsmittel, vorzugsweise NaN₃ aufweisen.

7. Verfahren nach einem oder mehreren der Ansprüche 1 bis 6, **dadurch gekennzeichnet, dass** als Harnstoffkonzentrat eine wässrige Lösung mit 8 bis 10 M, vorzugsweise 9,3 bis 9,8 M Harnstoff eingesetzt wird.

8. Verfahren nach einem der Ansprüche 1 bis 7, **dadurch gekennzeichnet, dass** wenigstens ein Pufferkonzentrat und das Harnstoffkonzentrat im Verhältnis 1 zu 3,7 gemischt werden.

9. *Verfahren nach einem oder mehreren der Ansprüche 2 bis 8, wobei die drei erzeugten Anwendungspuffer zur Bindung der Proteine an eine IMAC-Matrix, zum Waschen der IMAC-Matrix und zur Elution des interessierenden Proteins von der IMAC-Matrix eingesetzt werden.*

10. Verwendung eines Harnstoffkonzentrates zur Herstellung eines Anwendungspuffers mit definiertem pH-Wert, wobei eine definierte Menge eines Harnstoffkonzentrats mit einer definierten Menge eines Pufferkonzentrats mit voreingestelltem pH-Wert gemischt wird, wobei das Pufferkonzentrat keine störenden Mengen an Harnstoff aufweist.

11. Kit zur Aufreinigung von Proteinen über immobilisierte Metallaffinitätschromatographie (IMAC) unter denaturierenden Bedingungen, enthaltend wenigstens ein Pufferkonzentrat mit definiertem pH-Wert, wobei das Pufferkonzentrat keine störenden Mengen an Harnstoff aufweist, sowie wenigstens ein Harnstoffkonzentrat zur Herstellung eines Anwendungspuffers mit definiertem pH - Wert, wobei das Harnstoffkonzentrat eine wässrige Lösung mit 8 bis 10M Harnstoff ist und das Kit eine IMAC Matrix aufweist.

12. Kit nach Anspruch 11, enthaltend wenigstens drei Pufferkonzentrate mit unterschiedlichem, definierten pH-Wert, sowie ein Harnstoffkonzentrat zur Herstellung von wenigstens drei Anwendungspuffer mit jeweils unterschiedlichem, definiertem pH-Wert.

13. Kit nach Anspruch 11 oder 12, **gekennzeichnet durch** eines oder mehrere der folgenden Merkmale:
a. dass das Pufferkonzentrat, welches nach Mischung mit dem Harnstoffkonzentrat ein Anwendungspuffer zur Bindung ist, einen alkalischen pH-Wert aufweist; und/oder
b. dass das Pufferkonzentrat, welches nach Mischung mit dem Harnstoffkonzentrat ein Anwendungspuffer zur Bindung ist, einen pH-Wert von 7,0 bis 9,0 aufweist; und/oder
c. dass das Pufferkonzentrat, welches nach Mischung mit dem Harnstoffkonzentrat ein Anwendungspuffer zur Bindung ist, einen pH-Wert von ca. 7,5 aufweist; und/oder
d. dass das Pufferkonzentrat, welches nach Mischung mit dem Harnstoffkonzentrat ein Anwendungspuffer zum Waschen ist, einen leicht sauren bis neutralen pH-Wert aufweist; und/oder
e. dass das Pufferkonzentrat, welches nach Mischung mit dem Harnstoffkonzentrat ein Anwendungspuffer zum Waschen ist, einen pH-Wert von 5,5 bis 7,0 aufweist; und/oder
f. dass das Pufferkonzentrat, welches nach Mischung mit dem Harnstoffkonzentrat ein Anwendungspuffer zum Waschen ist, einen pH-Wert von ca. 5,6 aufweist; und/oder
g. dass das Pufferkonzentrat, welches nach Mischung mit dem Harnstoffkonzentrat ein Anwendungspuffer zur Elution ist, einen sauren pH-Wert aufweist; und/oder
h. dass das Pufferkonzentrat, welches nach Mischung mit dem Harnstoffkonzentrat ein Anwendungspuffer zur Elution ist, einen pH-Wert von unter 4, vorzugsweise unter 3,5 aufweist.

14. Kit nach einem oder mehreren der Ansprüche 11 bis 13, **dadurch gekennzeichnet, dass** das Pufferkonzentrat mit definiertem pH-Wert einen biologischen Puffer aufweist.

15. Kit nach einem der oder mehreren der Ansprüche 13 bis 14, **dadurch gekennzeichnet,**
a. **dass** das Pufferkonzentrat zur Herstellung des Binde-Anwendungspuffer einen Puffer aufweist, der eine Pufferkapazität im Alkalischen aufweist, vorzugsweise Tris; und
b. **dass** die Pufferkonzentrate zur Herstellung des Wasch- und **Elutionsanwendungspuffers** einen Puffer aufweisen, der eine Pufferkapazität im Neutralen bis Sauren aufweist, vorzugsweise Bis-Tris; und/oder
c. **dass** die Pufferkonzentrate Salze, vorzugsweise NaH₂PO₄, und/oder Detergenzien, vorzugsweise Tween und/oder ein Konservierungsmittel, vorzugsweise NaN₃ aufweisen.

16. Kit nach einem oder mehreren der Ansprüche 11 bis 15, **gekennzeichnet durch** eines oder mehrere der folgenden Merkmale:
a. das Kit weist eine Ni²⁺-NTA *Matrix* auf; und/oder
b. das Kit ist für die automatisierte Anwendung einsetzbar.

## Claims

1. Method for preparing an application buffer for the purification of proteins by immobilized metal affinity chromatography (IMAC) under denaturing conditions, **characterized in that** a defined amount of a buffer concentrate having a defined pH value that does not comprise interfering amounts of urea is mixed with a defined amount of a urea concentrate, whereby an application buffer having a defined pH value is provided.

2. Method according to claim 1, **characterized in that** at least three buffer concentrates each having a different pH value are mixed with a defined amount of a urea concentrate, whereby three application buffers are provided, each having a different, defined pH value.

3. Method according to claim 1 or 2, **characterized by** one or more of the following features:
a. that the buffer concentrate, which after mixing with the urea concentrate is an application buffer for binding, has an alkaline pH value; and/or
b. that the buffer concentrate, which after mixing with the urea concentrate is an application buffer for binding, has a pH value of from 7.0 to 9.0; and/or
c. that the buffer concentrate, which after mixing with the urea concentrate is an application buffer for binding, has a pH value of about 7.5; and/or
d. that the buffer concentrate, which after mixing with the urea concentrate is an application buffer for washing, has a slightly acidic pH value; and/or
e. that the buffer concentrate, which after mixing with the urea concentrate is an application buffer for washing, has a pH value of from 5.5 to 7.0; and/or
f. that the buffer concentrate, which after mixing with the urea concentrate is an application buffer for washing, has a pH value of about 5.6; and/or
g. that the buffer concentrate, which after mixing with the urea concentrate is an application buffer for elution, has an acidic pH value; and/or
h. that the buffer concentrate, which after mixing with the urea concentrate is an application buffer for elution, has a pH value of below 4, preferably below 3.5; and/or
i. the buffer concentrates do not comprise any urea.

4. Method according to any one of claims 1 to 3, **characterized by** one or more of the following features
a. that the application buffer for binding produced by mixing the buffer concentrate with the urea concentrate has an alkaline pH value; and/or
b. that the application buffer for binding produced by mixing the buffer concentrate with the urea concentrate has a pH value of from 7.0 to 9.0; and/or
c. that the application buffer for binding produced by mixing the buffer concentrate with the urea concentrate has a pH value of about 8; and/or
d. that the application buffer for washing produced by mixing the buffer concentrate with the urea concentrate has a slightly acidic to neutral pH value; and/or
e. that the application buffer for washing produced by mixing the buffer concentrate with the urea concentrate has a pH value of from 5.5 to 9.0; and/or
f. that the application buffer for washing produced by mixing the buffer concentrate with the urea concentrate has a pH value of from 6 to 6.5; and/or
g. that the application buffer for elution produced by mixing the buffer concentrate with the urea concentrate has an acidic pH value; and/or
h. that the application buffer for elution produced by mixing the buffer concentrate with the urea concentrate has a pH value of below 5.5, preferably below 5.

5. Method according to one or more of claims 1 to 4, **characterized in that** the buffer concentrate having a defined pH value comprises a biological buffer.

6. Method according to one or more of claims 3 to 5, **characterized in**
a. **that** the buffer concentrate for the preparation of the binding application buffer comprises a buffer having a buffer capacity in the alkaline range, preferably Tris; and
b. **that** the buffer concentrates for the preparation of the washing and elution application buffer comprise a buffer having a buffer capacity in the neutral to acidic range, preferably Bis-Tris; and/or
c. **that** the buffer concentrates comprise salts, preferably NaH₂PO₄ and/or detergents, preferably Tween and/or a preservative, preferably NaN₃.

7. Method according to one or more of claims 1 to 6, **characterized in that** as a urea concentrate an aqueous solution containing 8 to 10 M, preferably 9.3 to 9.8 M urea is used.

8. Method according to any one of claims 1 to 7, **characterized in that** at least one buffer concentrate and the urea concentrate are mixed in a ratio of 1 to 3.7.

9. Method according to one or more of claims 2 to 8, wherein the three prepared application buffers are used for binding the proteins to an IMAC matrix, for washing the IMAC-matrix and for eluting the protein of interest from the IMAC matrix.

10. Use of a urea concentrate for the preparation of an application buffer having a defined pH value, wherein a defined amount of said urea concentrate is mixed with a defined amount of a buffer concentrate having a pre-adjusted pH value, wherein the buffer concentrate does not comprise interfering amounts of urea.

11. Kit for purifying proteins by means of immobilized metal affinity chromatography (IMAC) under denaturing conditions, comprising at least one buffer concentrate having a defined pH value, wherein the buffer concentrate does not comprise interfering amounts of urea, and at least a urea concentrate for preparing an application buffer having a defined pH value, wherein the urea concentrate is an aqueous solution containing 8 to 10 M urea and the kit comprises an IMAC matrix.

12. Kit according to claim 11, comprising at least three buffer concentrates having a different, defined pH value and a urea concentrate for the preparation of at least three application buffers each having a different, defined pH value.

13. Kit according to claim 11 or 12, **characterized by** one or more of the following features:
a. that the buffer concentrate, which after mixing with the urea concentrate is an application buffer for binding, has an alkaline pH value; and/or
b. that the buffer concentrate, which after mixing with the urea concentrate is an application buffer for binding, has a pH value of from 7.0 to 9.0; and/or
c. that the buffer concentrate, which after mixing with the urea concentrate is an application buffer for binding, has a pH value of about 7.5; and/or
d. that the buffer concentrate, which after mixing with the urea concentrate is an application buffer for washing, has a slightly acidic to neutral pH value; and/or
e. that the buffer concentrate, which after mixing with the urea concentrate is an application buffer for washing, has a pH value of from 5.5 to 7.0; and/or
f. that the buffer concentrate, which after mixing with the urea concentrate is an application buffer for washing, has a pH value of about 5.6; and/or
g. that the buffer concentrate, which after mixing with the urea concentrate is an application buffer for elution, has an acidic pH value; and/or
h. that the buffer concentrate, which after mixing with the urea concentrate is an application buffer for elution, has a pH value of below 4, preferably below 3.5.

14. Kit according to one or more of claims 11 to 13, **characterized in that** the buffer concentrate having a defined pH value comprises a biological buffer.

15. Kit according to one or more of claims 13 to 14, **characterized in**
a. **that** the buffer concentrate for the preparation of the binding application buffer comprises a buffer having a buffer capacity in the alkaline range, preferably Tris; and/or
b. **that** the buffer concentrates for the preparation of the washing and elution application buffer comprise a buffer having a buffer capacity in the neutral to acidic range, preferably Bis-Tris; and/or
c. **that** the buffer concentrates comprise salts, preferably NaH₂PO₄, and/or detergents, preferably Tween and/or a preservative, preferably NaN₃.

16. Kit according to one or more of claims 11 to 15, **characterized by** one or more of the following features:
a. the kit comprises a Ni²⁺-NTA matrix; and/or
b. the kit is suitable for automated use.

## Revendications

1. Procédé pour la préparation d'un tampon d'utilisation pour la purification de protéines par chromatographie d'affinité métallique immobilisée (IMAC) dans des conditions de dénaturation, **caractérisé en ce qu'**on mélange une quantité définie d'un concentrat tampon, présentant un pH défini, qui ne présente pas de quantités gênantes d'urée, avec une quantité définie d'un concentrat d'urée, ce qui permet de mettre à disposition un tampon d'utilisation présentant un pH défini.

2. Procédé selon la revendication 1, **caractérisé en ce qu'**on mélange au moins trois concentrats tampon présentant à chaque fois un pH différent avec une quantité définie d'un concentrat d'urée, ce qui permet de mettre à disposition trois tampons d'utilisation présentant à chaque fois un pH défini, différent.

3. Procédé selon la revendication 1 ou 2, **caractérisé par** une ou plusieurs des caractéristiques suivantes:
a. le concentrat tampon, qui est, après mélange avec le concentrat d'urée, un tampon d'utilisation pour la liaison, présente un pH alcalin; et/ou
b. le concentrat tampon, qui est, après mélange avec le concentrat d'urée, un tampon d'utilisation pour la liaison, présente un pH de 7,0 à 9,0; et/ou
c. le concentrat tampon, qui est, après mélange avec le concentrat d'urée, un tampon d'utilisation pour la liaison, présente un pH d'environ 7,5; et/ou
d. le concentrat tampon, qui est, après mélange avec le concentrat d'urée, un tampon d'utilisation pour le lavage, présente un pH légèrement acide; et/ou
e. le concentrat tampon, qui est, après mélange avec le concentrat d'urée, un tampon d'utilisation pour le lavage, présente un pH de 5,5 à 7,0; et/ou
f. le concentrat tampon, qui est, après mélange avec le concentrat d'urée, un tampon d'utilisation pour le lavage, présente un pH d'environ 5,6; et/ou
g. le concentrat tampon, qui est, après mélange avec le concentrat d'urée, un tampon d'utilisation pour l'élution, présente un pH acide; et/ou
h. le concentrat tampon, qui est, après mélange avec le concentrat d'urée, un tampon d'utilisation pour l'élution, présente un pH inférieur à 4, de préférence inférieur à 3,5; et/ou
i. les concentrats tampon ne présentent vraiment aucune urée.

4. Procédé selon l'une quelconque des revendications 1 à 3, **caractérisé par** une ou plusieurs des caractéristiques suivantes
a. le concentrat d'utilisation pour la liaison, obtenu par mélange du concentrat tampon avec le concentrat d'urée, présente un pH alcalin; et/ou
b. le concentrant d'utilisation pour la liaison, obtenu par mélange du concentrat tampon avec le concentrat d'urée, présente un pH de 7,0 à 9,0; et/ou
c. le concentrat d'utilisation pour la liaison, obtenu par mélange du concentrat tampon avec le concentrat d'urée, présente un pH d'environ 8; et/ou
d. le concentrat d'utilisation pour le lavage, obtenu par mélange du concentrat tampon avec le concentrat d'urée, présente un pH légèrement acide à neutre; et/ou
e. le concentrat d'utilisation pour le lavage, obtenu par mélange du concentrat tampon avec le concentrat d'urée, présente un pH de 5,5 à 9,0; et/ou
f. le concentrat d'utilisation pour le lavage, obtenu par mélange du concentrat tampon avec le concentrat d'urée, présente un pH de 6 à 6,5; et/ou
g. le concentrat d'utilisation pour l'élution, obtenu par mélange du concentrat tampon avec le concentrat d'urée, présente un pH acide; et/ou
h. le concentrat d'utilisation pour l'élution, obtenu par mélange du concentrat tampon avec le concentrat d'urée, présente un pH inférieur à 5,5, de préférence inférieur à 5.

5. Procédé selon l'une ou plusieurs des revendications 1 à 4, **caractérisé en ce que** le concentrat tampon présentant un pH défini présente un tampon biologique.

6. Procédé selon l'une ou plusieurs des revendications 3 à 5, caractérisé
a. en ce que le concentrat tampon pour la préparation du tampon d'utilisation de liaison présente un tampon doté d'une capacité de tampon dans la plage alcaline, de préférence du Tris; et
b. en ce que les concentrats tampon pour la préparation d'un tampon d'utilisation pour le lavage et pour l'élution présentent un tampon doté d'une capacité de tampon dans la plage neutre à acide, de préférence du Bis-Tris; et/ou
c. en ce que les concentrats tampon présentent des sels, de préférence du NaH₂PO₄, et/ou ou des détergents, de préférence du Tween, et/ou un conservateur, de préférence du NaN₃.

7. Procédé selon l'une ou plusieurs des revendications 1 à 6, **caractérisé en ce qu'**on utilise, comme concentrat d'urée, une solution aqueuse contenant 8 à 10 M, de préférence 9,3 à 9,8 M d'urée.

8. Procédé selon l'une quelconque des revendications 1 à 7, **caractérisé en ce qu'**on mélange au moins un concentrat tampon et le concentrat d'urée dans un rapport de 1:3,7.

9. Procédé selon l'une ou plusieurs des revendications 2 à 8, les trois tampons d'utilisation obtenus étant utilisés pour la liaison des protéines à une matrice IMAC, pour le lavage de la matrice IMAC et pour l'élution de la protéine d'intérêt de la matrice IMAC.

10. Utilisation d'un concentrat d'urée pour la préparation d'un tampon d'utilisation présentant un pH défini, une quantité définie d'un concentrat d'urée étant mélangée avec une quantité définie d'un concentrat tampon présentant un pH préréglé, le concentrat tampon ne présentant pas de quantités gênantes d'urée.

11. Ensemble ou trousse pour la purification de protéines par chromatographie d'affinité métallique immobilisée (IMAC) dans des conditions de dénaturation, contenant au moins un concentrat tampon présentant un pH défini, le concentrat tampon ne présentant pas de quantités gênantes d'urée, ainsi qu'au moins un concentrat d'urée pour la préparation d'un tampon d'utilisation présentant un pH défini, le concentrat d'urée étant une solution aqueuse contenant 8 à 10 M d'urée et l'ensemble ou la trousse présentant une matrice IMAC.

12. Ensemble ou trousse selon la revendication 11, contenant au moins trois concentrats tampon présentant un pH défini, différent, ainsi qu'un concentrat d'urée pour la préparation d'au moins trois tampons d'utilisation présentant à chaque fois un pH défini, différent.

13. Ensemble ou trousse selon la revendication 11 ou 12, **caractérisé par** une ou plusieurs des caractéristiques suivantes:
a. le concentrat tampon, qui est, après mélange avec le concentrat d'urée, un tampon d'utilisation pour la liaison, présente un pH alcalin; et/ou
b. le concentrat tampon, qui est, après mélange avec le concentrat d'urée, un tampon d'utilisation pour la liaison, présente un pH de 7,0 à 9,0; et/ou
c. le concentrat tampon, qui est, après mélange avec le concentrat d'urée, un tampon d'utilisation pour la liaison, présente un pH d'environ 7,5; et/ou
d. le concentrat tampon, qui est, après mélange avec le concentrat d'urée, un tampon d'utilisation pour le lavage, présente un pH légèrement acide à neutre; et/ou
e. le concentrat tampon, qui est, après mélange avec le concentrat d'urée, un tampon d'utilisation pour le lavage, présente un pH de 5,5 à 7,0; et/ou
f. le concentrat tampon, qui est, après mélange avec le concentrat d'urée, un tampon d'utilisation pour le lavage, présente un pH d'environ 5,6; et/ou
g. le concentrat tampon, qui est, après mélange avec le concentrat d'urée, un tampon d'utilisation pour l'élution, présente un pH acide; et/ou
h. le concentrat tampon, qui est, après mélange avec le concentrat d'urée, un tampon d'utilisation pour l'élution, présente un pH inférieur à 4, de préférence inférieur à 3,5.

14. Ensemble ou trousse selon l'une ou plusieurs des revendications 11 à 13, **caractérisé en ce que** le concentrat tampon présentant un pH défini présente un tampon biologique.

15. Ensemble ou trousse selon l'une ou plusieurs des revendications 13 à 14, **caractérisé**
a. **en ce que** le concentrat tampon pour la préparation d'un tampon d'utilisation de liaison présente un tampon doté d'une capacité de tampon dans la plage alcaline, de préférence du Tris; et
b. **en ce que** les concentrats tampon pour la préparation d'un tampon d'utilisation pour le lavage et pour l'élution présentent un tampon doté d'une capacité de tampon dans la plage neutre à acide, de préférence du Bis-Tris; et/ou
c. **en ce que** les concentrats tampon présentent des sels, de préférence du NaH₂PO₄, et/ou ou des détergents, de préférence du Tween, et/ou un conservateur, de préférence du NaN₃.

16. Ensemble ou trousse selon l'une ou plusieurs des revendications 11 à 15, **caractérisé par** une ou plusieurs des caractéristiques suivantes
a. l'ensemble ou la trousse présente une matrice Ni²⁺-NTA; et/ou
b. l'ensemble ou la trousse est utilisable pour un emploi automatisé.
